# EUROPEAN PATENT SPECIFICATION

(11) **EP 4 507 554 B1**
(45) Date of publication and mention of the grant of the patent: **18.02.2026**
(21) Application number: 23716935.4
(22) Date of filing: 28.03.2023
(51) Int. Cl.: A61B 1/00, A61B 1/31, A61B 1/015, A61M 25/01

(54) **ENDOSCOPIC GUIDE, IN PARTICULAR FOR COLONOSCOPES, AND ENDOSCOPY SYSTEM COMPRISING SAID GUIDE**
ENDOSKOPISCHE FÜHRUNG, INSBESONDERE FÜR KOLONOSKOPE, UND ENDOSKOPIESYSTEM MIT DIESER FÜHRUNG
GUIDE ENDOSCOPIQUE, DESTINÉ EN PARTICULIER AUX COLONOSCOPES, ET SYSTÈME D'ENDOSCOPIE COMPRENANT LEDIT GUIDE

(30) Priority: 14.04.2022 IT 202200007448
(43) Date of publication of application: 19.02.2025
(73) Proprietor: Endostart S.r.l., 50052 Certaldo, Firenze (IT)
(72) Inventor: TOZZI, Alessandro, 50052 CERTALDO, FIRENZE (IT); SANGREGORIO, Claudio, 50052 CERTALDO, FIRENZE (IT); TUCI, Tommaso, 50052 CERTALDO, FIRENZE (IT); MASIELLO, Fabiana, 50052 CERTALDO, FIRENZE (IT); MONDELLO, Giorgia, 50052 CERTALDO, FIRENZE (IT); LAZZERI, Denise, 50052 CERTALDO, FIRENZE (IT); ALBINO, Martin, 50052 CERTALDO, FIRENZE (IT); PETRECCA, Michele, 50052 CERTALDO, FIRENZE (IT)
(74) Representative: Long, Giorgio
(86) International application number: PCT/IB2023/053075
(87) International publication number: WO 2023/199143

(56) References cited:
- EP-B1- 3 399 901
- WO-A2-02/07794
- US-A1- 2020 113 416

## Description

The present invention relates to an endoscopic guide for catheters or endoscopes, particularly suitable for application in colonoscopy.

Examples of such instruments are colonoscope, enteroscope, gastroscope, duodenoscope, echoendoscope, and the like.

### Background art

Endoscopic guides (or "guide wires" in technical jargon) are used in the field to position and act as a guide, in particular for a catheter, which is normally mounted sliding thereon; in this regard, the catheter is provided with an operating channel in which the guide itself is placed in assembled condition.

Various types of endoscopic guides are known from the prior art, according to the applications they are intended for.

EP3399901B1 describes the preamble of claim 1.

A type of endoscopic guide provides an anchoring head which allows the guide to remain in place and act as a support for the operations of the catheter and endoscope.

In this regard, in certain embodiments the guides are provided with an inflatable balloon which, once introduced into the lumen of the organ and positioned, is inflated with air up to interfering with the walls of the lumen itself, thus remaining blocked in place.

Although these guides with an expandable anchoring head are used, they have considerable drawbacks.

Another limit of some of these solutions is associated with the fact that the lumen in which the guide head is positioned is occluded: in particular, in the case of inflatable balloons, the occlusion is complete.

Moreover, such anchoring heads are difficult to use in certain fields of endoscopy, such as in the case of colonoscopy, for example, due to the anatomical shape of the organ in which the guide is introduced. Indeed, the diameter of the lumen in the colon can vary from 2-3 cm up to 6-8 cm in relation to the segment considered and the presence or lack of pathological conditions or anatomical anomalies. This would result in the need for even quite bulky balloons.

Moreover, the anchoring via the balloons is obtained by virtue of the friction generated between the balloon and the surface of the organ mucosa. This may result in lesions to the organ walls. In the case of colonoscopy, the friction blockage results in the need to increase the pressure on the wall of the viscera, stretching out the balloon more, to obtain more stable anchoring. However, this operation could result in the excessive stretching of the viscera, with the risk of causing pain or even laceration of the tissue. Moreover, at times, especially in the elderly or in certain pathological conditions, the viscera can be dilated or flaccid, to the point of providing no resistance to the balloon dilation. In this condition, not only can the stretching of the balloon be risky, but also ineffective.

However, obtaining a firm and stable anchoring is necessary in order to ensure that the guide can also operate for colonoscopes, and endoscopes in general.

In particular, the colonoscopy technique requires care in introducing the colonoscope up to the end point of the colon, called the caecum, because the colon has a winding path and flaccid walls, whereby loops preventing the progression of the colonoscope tip and stretching the walls of the colon are formed, resulting in the occurrence of pain and the risk of laceration of the viscera: in these cases, the physician must maneuver the instrument in an adequate manner so as to rectify the path as much as possible and proceed by introducing the colonoscope up to the caecum or in any case exploring the entire viscera.

It should also be noted that the main difference between a colonoscope and a standard catheter is the greater mass of the first. A guide with increased rigidity is required as the mass of the catheter or endoscope increases. However, the guides currently being used in endoscopy cannot be made of particularly rigid material because the rigid tip could damage the viscera.

Many problems associated with the devices of the prior art have been overcome by an endoscopy guide and an endoscopy system comprising it as described in European Patent EP3399901A1 to the Applicant. Such a patent describes a system for magnetically anchoring the tip of the endoscopy guide by introducing a magnetic fluid consisting of an aqueous suspension of iron powder, magnetite, maghemite or ferrofluid into an inflatable balloon. The anchoring is obtained by applying, from the outside, a magnetic field having adequate force.

Although such a system allows obtaining an adequate anchoring to the wall of the colon without obstructing the lumen of the organ and without damaging the tissue thereof, it has various drawbacks.

A first problem is that during the introduction step along the winding path of the colon, the guide catheter is likely to experience the so-called kinking phenomenon.

It was also found that by using the volumes of magnetic fluid provided in the preceding patent and the concentration of magnetizable particles contained therein, an excessive viscosity of the fluid was obtained: this resulted in lengthy times for filling and emptying the balloon, and in an excessive force that the operator needed to apply for the filling.

Additionally, the frequent formation of clusters and/or masses of ferromagnetic particles in the balloon after the application of the magnetic field further slowed down or even prevented the emptying at the end of the operation.

The same clusters and/or masses of ferromagnetic particles in certain cases already formed in the pre-filled syringe during storing, thus preventing the filling of the balloon and resulting in the unusability of the system arranged for the latter.

Thus, it is a task of the invention to provide an endoscopic guide for catheters or endoscopes which solves one or more of the technical problems set out above with respect to the known magnetic anchoring system.

In particular, it is an object of the invention to manufacture an endoscopic guide which is quicker to operate with less effort or risk of blocking as compared to the known endoscopic guide with magnetic anchoring, while being relatively affordable and simple to manufacture, so as to be a disposable system.

### Summary of the invention

One or more of the tasks and objects set out above and others which can be apparent below are achieved by:
1) an endoscopy system comprising an endoscopic guide, in particular for colonoscopes, comprising a tubular guide element and an anchoring head, where the tubular guide element comprises a longitudinal cavity, the anchoring head being provided with at least one expandable container configured to accommodate a ferromagnetic agent, the expandable container being in communication with said longitudinal cavity, where the endoscopy system further comprises a ferromagnetic agent configured to be movable in said longitudinal cavity to fill/empty the container, characterized in that said ferromagnetic agent is an aqueous suspension of carbonyl iron.

Other objects of the invention are defined by the appended claims.

The invention further relates to an endoscopy system, preferably for colonoscopy, as defined in the appended claims, comprising an endoscopic guide according to the invention, acting in conjunction with an external magnetic field source, where the magnetic field source is preferably associated with a manually maneuverable device, i.e., also without movement apparatuses or assisted support. In certain embodiments, the manually maneuverable device is a handpiece in which the magnetic field source is movable between an operating position and a safety or resting position, where:
in the operating position, the magnetic field source is close to the operating end of the handpiece,
in the safety or resting position, the magnetic field source is far from the operating end of the handpiece.

Further features and advantages will become apparent from the description of preferred, but not exclusive, embodiments of the invention, illustrated by way of non-limiting example.

### Brief description of the drawings

Figure 1A is a sectional view of a first embodiment of a syringe for the ferromagnetic fluid according to the invention;
Figure 1B is a side view according to direction A of the syringe in Figure 1A;
Figure 2 is a sectional view of a detail of the syringe in Figure 1A;
Figure 3 is a sectional view of a different detail of the syringe in Figure 1A;
Figures 4A and 4B are perspective and plan views, respectively, of another different detail of the syringe in Figure 1A;
Figure 5 is a longitudinal sectional view of the piston of the syringe in Figure 1A;
Figure 6 is a cross-section view of the tubular guide element of the system according to the invention;
Figure 7 is a side view of the expandable container of the system according to the invention, in expanded condition.

### Detailed description of the invention

Firstly, it is useful to describe how a diagnostic colonoscopy examination is performed to better understand the invention.

The colonoscope is a flexible endoscope characterized by a tip (about 15 cm long), a body (about 130 cm long), and a manipulator. The tip is movable and accommodates the camera, the lights and the orifice of the operating channel therein. The tip can be maneuvered by moving the knobs located on the manipulator. The body has a flexibility which, in certain models, can also be selected for performing certain endoscopic maneuvers. The cables for transmitting the images, the electric supply and the tie rods for connecting the tip to the knobs slide inside the body.

In addition to the knobs for orienting the tip, the manipulator is also characterized by the external orifice of the operating channel and by the controls for drawing or blowing air and recording images or videos.

The diagnostic colonoscopy aims to inspect the whole surface of the colon mucosa so as to identify any pathological alterations thereof which possibly require further diagnostic or therapeutic interventions. It is necessary for the colonoscope tip provided with cameras and lights to reach the last part of the colon, referred to as the caecum. If indeed the instrument were not to reach the caecum, the physician could not affirm whether there are any pathological alterations in the unexplored segment.

The positioning of the colonoscope is performed by an endoscopic guide which is moved forward into the lumen of the colonoscope up to stretching out from the distal end thereof and advancing as deeply as possible, to then guide the path of the colonoscope. A similar technique is performed to introduce other endoscopes, as well as to introduce catheters into the vein system.

The colon is a movable hollow, tube-like organ with loose walls. Unlike other hollow, tube-like organs, such as arteries, for example, it has a larger diameter, the walls can stretch out over several centimeters, and in certain segments, the viscera can be mobilized in the abdominal cavity, at times by up to 30 cm.

Such anatomical features prevent the insertion of catheters or guides over segments of more than 10-30 cm. Indeed, once inserted into the rectum via the anus, catheters tend to coil up on themselves due to the large diameter of the lumen and the curves of the viscera. Therefore, unlike what is done in the vascular field, the catheterization of the colon is difficult to perform, unless over a short segment. A catheter or an endoscopy guide do not have adequate mobility of the tip or adequate bearing strength even during a radiological checkup. Bearing strength of an endoscope, catheter, or endoscopic guide, means the capacity to transmit the thrust from the body of the instrument to the tip thereof. The greater the rigidity of an endoscope, the greater the bearing strength. However, a too rigid endoscope cannot be easily maneuvered along the winding path of the colon. The colonoscope is designed so as to be a compromise between adequate flexibility and adequate bearing strength.

However, bearing strength progressively decreases if the colonoscope is in an excessively flexed arrangement. For example, if the colonoscope forms a loop during the colonoscopy, it can no longer be capable of transmitting the thrust from the body to the tip, and therefore the endoscopist is not able to further advance the instrument and the camera thereof. In these cases, the endoscopist manually pushing the instrument only results in the transmission of the force on the walls of the viscera, resulting in pain and the risk of trauma, moreover without advancing the tip.

In order to solve this drawback, the endoscopic guide suggested in the above-mentioned Patent EP3399901A1 to the Applicant allows performing a non-claimed method for introducing the colonoscope comprising the following steps:
- introducing, via the patient's anus, the distal end of the colonoscope up to reaching a blocking point, i.e., a point beyond which the colonoscope can no longer be advanced;
- advancing the endoscopic guide up to a distance of 4-10 cm past the distal end of the colonoscope;
- introducing, into the expandable container of the endoscopic guide, a magnetic or ferromagnetic agent so that the container reaches an expanded condition;
- applying an external magnetic field by means of a magnetic field source at the area of the abdomen in which there is the expanded container with the magnetic or ferromagnetic agent so as to attract the container against an anchoring point on the wall of the colon with a retaining force on the magnetic or ferromagnetic agent, with respect to a transverse pulling direction, at least equal to or greater than 5 Newton;
- tensioning the endoscopic guide by applying a pulling force of at least 5 Newton on the proximal end of the endoscopic guide;
- advancing the colonoscope up to the anchoring point of the container;
- repeating the above steps up to reaching an anchoring point at the caecum.

Pulling the endoscopic guide aims to keep the guide tensioned, allowing an easier sliding of the endoscope, and to at least partially reduce the curvature of the loops of the colon, even in this case in order to promote the introduction of a relatively rigid endoscope.

As mentioned above, when performing the above-described method, the endoscopy system described in EP3399901A1 has underlined certain criticalities which have made the use thereof poorly applicable to a diagnosis. Thus, the present invention results from the need to overcome such criticalities.

The system according to the invention will now be described, even with reference to the drawings.

The endoscopy system according to the invention comprises an endoscopic guide, in particular for colonoscopes, comprising a tubular guide element 1 and an anchoring head 2, where the tubular guide element 1 comprises a longitudinal cavity 3, the anchoring head 2 being provided with an expandable container 4 configured to accommodate a ferromagnetic agent, the expandable container 4 being in communication with said longitudinal cavity 3, where the endoscopy system further comprises a ferromagnetic agent configured to be movable in said longitudinal cavity to fill/empty the expandable container 4.

In preferred embodiments, the system further comprises a syringe 5 pre-filled with said ferromagnetic agent, or a syringe pre-filled with saline solution, and a vial with a ferromagnetic agent as a powder for the reconstitution of the mixture prior to the endoscopic procedure, or a vial with a suspension of ferromagnetic agent and an empty syringe, which is filled prior to the endoscopic procedure by drawing the mixture from a glass vial.

The tubular guide element 1 comprises a proximal end 1a, proximal to an operator, and a distal end 1b.

The anchoring head 2 can be placed at or adjacent to the distal end 1b of the tubular guide element 1, while a connecting device 6, preferably removably coupled to the tubular guide element 1, is placed at the proximal end 1a. The connecting device 6 is configured to be removably coupled to said pre-filled syringe 5.

According to the invention, the tubular guide element 1 has at least one longitudinal cavity 3 and the anchoring head 2 is provided with an expandable container 4 for accommodating said ferromagnetic agent.

The tubular guide element 1 preferably has a single lumen (i.e., it houses only one longitudinal cavity 3 therein), but in some variants, it has further cavities (for example, two cavities in the case of dual lumen) for the passage of air, fluids or endoscopic accessories; in this case, it can comprise a sheath defining each further lumen, which sheath can end past the anchoring head 2.

It is important for the endoscopic guide to have a sufficiently wide lumen so as to allow the ferromagnetic agent to quickly and easily flow, while simultaneously having reduced kinking. In this regard, the tubular guide element 1 of the endoscopic guide of the invention preferably has an outer diameter D less than 2.6 mm and an inner diameter d of the lumen of the tubular guide element 1 between 1.6 mm and 1.75 mm.

In particularly preferred embodiments, the tubular guide element 1 has an outer diameter of about 2.5 mm, an inner diameter of about 1.7 mm, and therefore a thickness of wall S of about 0.4 mm. This sizing is particularly advantageous both in terms of reduced kinking and for the insertion thereof even into flexible optical endoscopes having a diameter from 3.2 mm. The term "about" means a dimensional variation more or less within the usual measuring errors or production variability.

Instead, as for the overall length of the tubular guide element 1, it is preferably such as to allow the replacement, during the process, of a catheter with another catheter, for example a different type of catheter. Generally, the tubular guide element 1 has a length between 2.5 and 4 meters.

The expandable container 4 is placed in communication with the longitudinal cavity 3 of the tubular guide element 1.

In the preferred embodiment, the mutual arrangement of container 4 and the tubular guide element 1 is as illustrated, i.e., the expandable container 4 is placed close to the distal end of the tubular guide element 1, which crosses the expandable container 4 and projects therefrom with an end portion 1c.

The length of the end portion 1c varies from a few millimeters to several centimeters; in the second case, it is preferable for it to be soft and flexible so as not to damage the mucosa if the guide is pushed towards the wall of the viscera.

In this embodiment, the expandable container 4 thus extends peripherally around the tubular guide element 1 to surround it completely at least over a given axial length.

In other embodiments (not shown), the expandable container 4 is instead placed to cover the distal end 1b of the tubular guide element 1, which leads into the expandable container 4.

The expandable container 4 is manually expandable and retractable, preferably in an elastic manner, so as to switch from a retracted condition, in which it substantially adheres to the body of the tubular guide element 1, to an expanded condition, in which it projects externally from the body of the tubular guide element 1, and vice versa; the filling or emptying of the expandable container 4 by means of the ferromagnetic agent results in the switching from the retracted position to the expanded one, and vice versa.

The expandable container 4 comprises, or alternatively consists of, an inflatable balloon. Inflatable balloons can be elastic, semi-elastic or inelastic. Examples of inflatable balloons are those normally used in endoscopy, for example nylon balloons or balloons made of a polyether-polyamide block copolymer, or a compound thereof with a polyamide. Such copolymers and the compounds thereof with polyamides are known and were described in detail in Patent Publication WO 2007/132485 A1.

In preferred embodiments, the expandable container 4 is sized so as to have a capacity of 22-28 ml, more preferably of about 25 ml, and when in expanded condition, a length between 55 and 65 mm, more preferably of about 60 mm, and a transversal diameter between 20 and 30 mm, more preferably of about 25 mm. Such dimensions allow not occluding the lumen of the colon, which has a varying diameter between 3 and 7 cm, depending on the cross section observed.

Optionally, the tubular guide element 1 comprises a valve arranged upstream of the expandable container 4, i.e., in a position more proximal to the operator, adapted to prevent, when the expandable container 4 is filled with the ferromagnetic agent, the latter from flowing back.

After lengthy and accurate experimentation, the Applicant has found that the ferromagnetic agent most suited to the purposes of the invention is an aqueous suspension of so-called carbonyl iron.

Carbonyl iron is a highly pure iron (97.5% for grade S, at least 99.5% for grade R), prepared by chemical decomposition of purified iron pentacarbonyl. It usually has the aspect of a grey powder consisting of spherical micro particles. Most of the impurities are carbon, oxygen and nitrogen. The most common uses are in powder metallurgy, metal injection molding and in various special products. Carbonyl iron powder is used in pharmaceutical applications to treat iron deficiency and as an iron food supplement due to the low toxicity thereof.

For the purposes of the present invention, carbonyl iron preferably having the following particle size profile is used:
D10= 3-4.5 microns, more preferably about 4.1 microns
D50= 5-10 microns, more preferably about 9.5 microns
D90= 4-30 microns, more preferably about 26 microns.

The particle size of the ferromagnetic particles can be determined with known methods, such as:
- direct observation by electronic microscopy;
- determination with methods of "laser light scattering" on the aqueous suspension using a particle size laser analyzer (Malvern Mastersizer 3000) with LALLS (Low Angle Laser Light Scattering) technique using the Fraunhofer calculation theory.

The aqueous suspension of carbonyl iron according to the invention preferably is a suspension in purified water in which the carbonyl iron is present in an amount by weight between 45% and 60%, preferably between 50% and 55%, more preferably between 52% and 53%, with respect to the total weight of the suspension. Preferably, said aqueous suspension further comprises tribasic sodium citrate in amounts by weight between 2.5% and 4.5% with respect to the total weight of the suspension, and/or sodium chloride in amounts by weight between 5% and 8% with respect to the total weight of the suspension.

The tribasic sodium citrate promotes the dispersion of the particles of carbonyl iron in the suspension.

The sodium chloride serves to inhibit bacterial proliferation.

The aqueous suspension of carbonyl iron is preferably degassed, for example by insufflation of an inert gas such as nitrogen or argon, so as to avoid oxidative processes of the iron by the oxygen absorbed in water.

The syringe preferably is vacuum packaged with atmosphere modified with an inert gas such as nitrogen or argon. The bags used have an aluminum coating in order to limit the passage of oxygen.

It has been noted that the use of the aqueous suspension of carbonyl iron according to the present invention results in several advantages as compared to the known solutions, i.e.:
i) it has an increased magnetic response, whereby the quantity of aqueous dispersion used, and therefore the filling volume of the expandable container 4, can be decreased;
ii) the spherical shape of the particles results in less viscosity of the aqueous suspension and reduces the risk of the formation of clusters and/or masses;
iii) carbonyl iron is widely available on the market, has a moderate cost and a low toxicity, is compatible with a pharmaceutical use.

Many problems emerged with the known endoscopy system are thus solved with the use of the aqueous suspension of carbonyl iron according to the invention, and in particular an increased speed for filling and emptying the expandable container 4 is obtained (due to the lower viscosity of the suspension and smaller amount of suspension required, the force of attraction being equal), less effort by the operator (lower viscosity of the suspension and smaller volume to be pumped with the syringe), less risk of kinking of the tubular guide element 1 (smaller inner diameter allowed by the lower viscosity and smaller amount of aqueous suspension of ferromagnetic agent required).

The endoscopy system of the invention comprises an endoscopic guide as described above and further comprises an external magnetic field source configured to apply a retaining force to the ferromagnetic agent, with respect to a transverse pulling direction, which is greater than 5 Newton at a distance between 2 centimeters and 10 centimeters.

The term "apply a retaining force to the ferromagnetic agent with respect to a transverse pulling direction" means the retaining force to the ferromagnetic agent with respect to a pulling substantially perpendicular to the direction of maximum attraction between magnet and ferromagnetic body, as determined by the lines of force of the magnetic field.

The retaining force of the expandable container 4 against the wall of the colon, with respect to a transverse pulling direction which, as mentioned, is to be equal to or greater than 5 Newton to allow adequate pulling of the endoscopic guide, depends both on the amount of ferromagnetic agent in container 4 and on the magnetic field applied, which in turn depends on the distance at which the source of the magnetic field is from the container 4 filled with the ferromagnetic agent. Usually, such a distance is between 2 and 5 cm in most colon segments. However, such a distance is greater in obese patients, for example up to 10 cm.

The external magnetic field source is selected as a function of the type of use of the instrument and the type of patient. A permanent magnet or an electromagnet can be used, the first being preferred. For the purposes of the invention, it is possible to use a magnetic field equal to that generated by a permanent magnet with discoidal magnetization N45 (i.e., a magnet in which the maximum magnetic energy per volume storable in the magnet is 45 Mega-Gauss-Oersted) having diameter between 50 and 80 mm and thickness between 30 and 60 mm. If a permanent magnet is used, it can be made of sintered neodymium, sintered ferrite, plasto-neodymium, or the like.

The magnetic field source can preferably be contained in a handpiece specifically adapted for this application, such as that described in EP3399901A1.

In preferred embodiments, the step of applying the magnetic field can be performed by applying such a magnetic field in a progressive manner. If indeed the required magnetic field were applied quite quickly, it would cause a sudden movement of container 4, which would then cause a sudden and painful stretching of the patient's viscera. The progressive application of the magnetic field can be obtained in two ways:
A) increasing the magnetic field applied by progressively varying the distance of the external magnetic field source, or
B) progressively introducing aliquots of ferromagnetic agent into the expandable container 4 with external magnetic field already applied.

As can be understood, as much as the use of a handpiece specifically adapted for this application is preferable, it is not indispensable for the correct use of the endoscopic guide of the invention. Indeed, the magnetic field source can be positioned in a plate or other instrument fixed above the bed on which the patient is, or movable above it in a manual or automatically controlled manner. For example, the permanent or electromagnetic magnetic field source can be connected to a movable arm, for example a jointed arm, which can be brought to the operating condition by maneuvering it manually or controlling the movements thereof by means of electric actuators or even automatically, based on a preselected operating program.

As mentioned, the introduction of the ferromagnetic agent occurs by means of a syringe. The emptying of container 4 can also be obtained in the same manner.

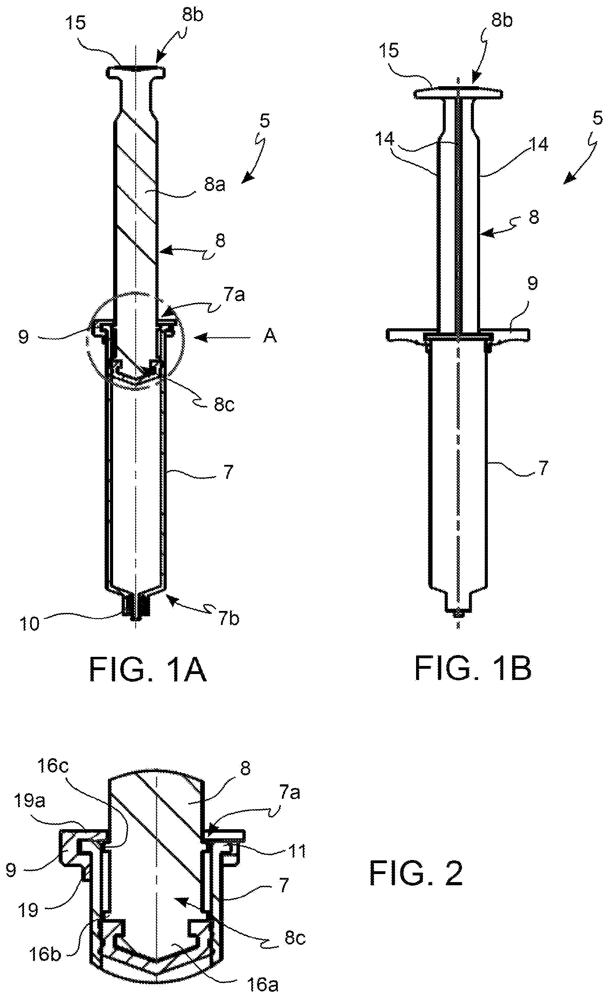

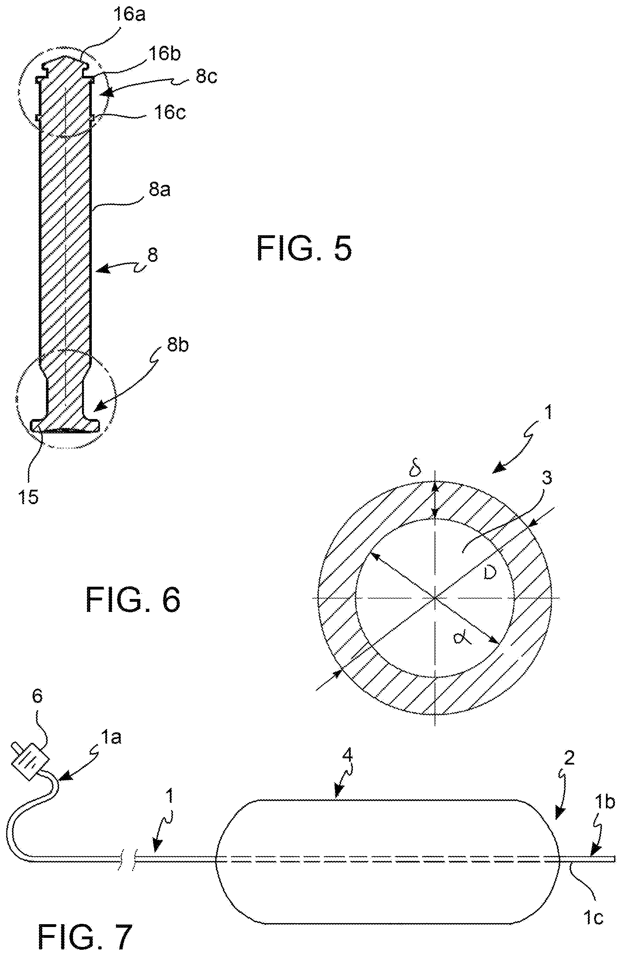

A syringe specifically adapted to the purposes of the present invention is shown in Figures 1A to 5.

Such a syringe, indicated as a whole by reference numeral 5, comprises a cylinder 7 in which a piston 8 is slidingly inserted, and a removable gripping flange 9.

Cylinder 7 comprises an open end 7a, in which the piston 8 is insertable, and a joining end 7b, opposite to the open end 7a, provided with a connector 10 with the connecting device 6 of the tubular guide element 1.

The open end 7a of cylinder 7 comprises an outwardly projecting annular shoulder 11.

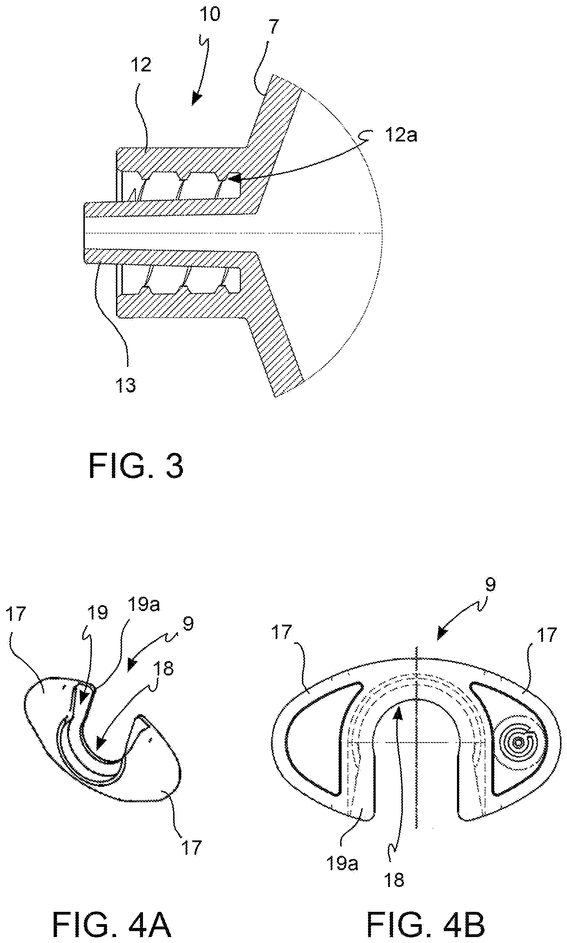

Connector 10, shown in detail in Figure 3, preferably is of the male "Luer lock" type and comprises a cylindrical sleeve 12 having an inner thread 12a and a nozzle 13 arranged coaxially inside the cylindrical sleeve 12.

Cylinder 7 has a greater total volume than the filling volume (i.e., the volume occupied by the ferromagnetic agent) so as to facilitate the emptying operation. Preferably, when the filling volume is about 25 ml, the total volume is about 35 ml.

Piston 8 comprises a body 8a developing along a longitudinal axis between a proximal portion 8b and a distal portion 8c, and preferably consists of a structure 14 with flaps arranged in a cross so as to decrease the friction with the inner walls of cylinder 7 and form a rigid structure.

The proximal portion 8b comprises a thrust button 15 of sufficiently ample dimensions so as to allow the operator to impart adequate pressure. For example, the thrust button 15 can have the dimensions 40 mm x 25 mm.

The distal portion 8c comprises a tip 16a, a sealing flange 16b and a stop flange 16c. When syringe 5 is pre-filled with the ferromagnetic agent and is assembled, the stop flange 16c is placed level with the annular shoulder 11 of cylinder 7, thus determining the thrust position of piston 8.

The gripping flange 9, shown in Figures 4A and 4B, has, for example, an elliptic plan shape and comprises flaps 17 which are placed alongside a recess 18.

The gripping flange 9 also comprises a groove 19 surrounding recess 18 and has a portion 19a projecting towards the interior of recess 18.

The gripping flange 9 is assembled to the pre-filled syringe by inserting recess 18 at the open end 7a of cylinder 7 so that the annular shoulder 11 is inserted in groove 19.

As shown in Figure 2, in addition to forming a gripping surface for an operator's index and middle finger during the operation of the syringe with the thumb placed on the thrust button 15, the gripping flange 9 also allows piston 8 to be retained, preventing the removal thereof from cylinder 7, by virtue of the abutment between the stop flange 16c of cylinder 7 and the projecting portion 19a of the gripping flange 9.

The gripping flange 9 has sufficiently ample dimensions so as to allow the operator a comfortable and secure grip. For example, the gripping flange can have the dimensions 65 mm x 36 mm.

Syringe 5 can be made, for example, of polycarbonate (for example, a material referred to as Makrolon^{®}) or cyclic olefin (co)polymers (referred to with the acronym COP and COC, respectively).

The invention further relates to an endoscopy kit, comprising an endoscopic guide according to the invention, a syringe 5 pre-filled with an aqueous suspension of carbonyl iron in a preset amount, and optionally a catheter.

Other variants of the kit according to the invention are:
- a kit comprising a glass vial filled with the powdered ferromagnetic agent and a pre-filled syringe filled with saline solution (aqueous solution of NaCl and/or sodium citrate), or
- a kit comprising a glass vial with the ferromagnetic agent suspension and an empty syringe.

In certain embodiments of the kit of the invention, syringe 5 is preloaded with a suspension of said carbonyl iron, preferably in a volume between 20 and 30 ml, more preferably about 25 ml.

In essence, it has been found how the invention achieves the preset tasks and objects because it allows providing an endoscopic guide, in particular for colonoscopes, which allows performing the diagnostic analysis or the surgical intervention of removing a lesion of the colon in a quick and complete manner, by virtue of the fact that the colonoscope can be inserted up to the maximum point at the caecum by tensioning the guide. Such a tensioning is allowed by the strong anchoring obtainable with the guide of the invention, which in turn is the result of the considerable amount of ferromagnetic agent which can be introduced into the expandable container 4 and the increased magnetic field applicable from the outside by means of the handpiece or other magnetic or electromagnetic field source.

Moreover, the endoscopic guide allows performing at least small movements of the anchoring head 2 even when it is in blocking position: indeed, in this regard, the operator can use the external magnetic field to move the anchoring head to an appropriate position.

Moreover, the operator has an available endoscopic guide for which it is sufficient to rest in a limited area of the lumen of the organ in which it is introduced to ensure a stable fastening, therefore without the need to occlude the whole lumen.

Not lastly, it has been found that the endoscopic guide and the system comprising such a guide are relatively quick to operate, in addition to being relatively affordable and simple to manufacture. The risk of kinking of the endoscopic guide is significantly reduced and the viscosity of the aqueous suspension of carbonyl iron is sufficiently low so as to allow quick times for filling and emptying the expandable container 4 and a limited effort by the operator. The risk of the formation of clusters and/or masses of ferromagnetic particles is also low.

Finally, the guide and the system are relatively safe also in case of malfunctioning when performing the examination. The aqueous suspension of carbonyl iron has a low toxicity and is already used in medical field as an iron supplement. This excludes risks for the patient in the case of loss of the expandable container 4.

A further advantage of the invention is associated with the possibility of a more accurate localization of a lesion, for example in the case of colonoscopy: indeed, the point where a lesion of the colon is found can be localized (topographically) on the patient's abdomen. Indeed, in use, the external handpiece is magnetically coupled to the guide head in the point of the abdominal surface closest to the anchoring head, which can advantageously be used to highlight where a lesion is with respect to the abdominal surface.

It is apparent that only some particular embodiments of the present invention have been described, to which those skilled in the art will be able to make all changes required to adapt it to particular applications, without departing from the scope of protection of the present invention.

In practice, the materials used, as long as they are compatible with the specific use, as well as the contingent dimensions and shapes, can be varied according to the requirements and advancement of the art.

## Claims

1. An endoscopy system, in particular for colonoscopy, comprising:
- an endoscopic guide, in particular for colonoscopes, comprising a tubular guide element (1) and an anchoring head (2), wherein the tubular guide element (1) comprises a longitudinal cavity (3), the anchoring head (2) being provided with an expandable container (4) configured to accommodate a ferromagnetic agent, the expandable container (4) being in communication with said longitudinal cavity (3), wherein the endoscopy system further comprises a ferromagnetic agent configured to be movable in said longitudinal cavity to fill/empty the expandable container (4),
**characterized in that** the ferromagnetic agent is an aqueous suspension of carbonyl iron.

2. The endoscopy system according to claim 1, further comprising a magnetic field source configured to apply a retaining force to the ferromagnetic agent, with respect to a transverse pulling direction, which is greater than or equal to 5 Newton at a distance between 2 centimeters and 10 centimeters from said ferromagnetic agent.

3. The system according to claim 1 or 2, further comprising a syringe (5) pre-filled with said ferromagnetic agent.

4. The system according to any one of claims 1 to 3, wherein said carbonyl iron has a purity greater than or equal to 97.5%, or greater than or equal to 99.5%, and consists of spherical particles having the following particle size profile:
D10= 3-4.5 microns, or about 4.1 microns
D50= 5-10 microns, or about 9.5 microns
D90= 4-30 microns, or about 26 microns.

5. The system according to any one of claims 1 to 4, wherein said aqueous suspension of carbonyl iron comprises an amount by weight of carbonyl iron between 45% and 60%, or between 50% and 55%, or between 52% and 53%, with respect to the total weight of the suspension.

6. The system according to any one of claims 1 to 5, wherein said aqueous suspension of carbonyl iron comprises tribasic sodium citrate in amounts by weight between 2.5% and 4.5% with respect to the total weight of the suspension and/or sodium chloride in amounts by weight between 5% and 8% with respect to the total weight of the suspension.

7. The system according to any one of claims 1 to 6, wherein the tubular guide element (1) has a single lumen and has an outer diameter (D) less than 2.6 mm, or of about 2.5 mm, and an inner diameter (d) of the lumen of the tubular guide element (1) between 1.6 mm and 1.75 mm, or of about 1.7 mm.

8. The system according to any one of claims 1 to 7, wherein the expandable container (4) comprises, or consists of, an inflatable balloon and is sized so as to have a capacity of 22-28 ml, or of about 25 ml, and when in expanded condition, a length between 55 and 65 mm, or of about 60 mm, and a transversal diameter between 20 and 30 mm, or of about 25 mm.

9. The system according to claim 3, or any one of claims 4 to 8 when dependent on claim 3, wherein the syringe (5) comprises a cylinder (7) in which a piston (8) is slidingly inserted, and a removable gripping flange (9).

10. The system according to claim 9, wherein the cylinder (7) comprises an open end (7a), in which the piston (8) is insertable, and a joining end (7b), opposite to the open end (7a), provided with a connector (10) for the tubular guide element (1), wherein the open end (7a) comprises an outwardly projecting annular shoulder (11) and wherein the connector (10) is of the male "Luer lock" type and comprises a cylindrical sleeve (12) having an inner thread (12a) and a nozzle (13) arranged coaxially inside the cylindrical sleeve (12).

11. The system according to claim 9 or 10, wherein the piston (8) comprises a body (8a) developing along a longitudinal axis between a proximal portion (8b) and a distal portion (8c), and preferably consists of a structure (14) with flaps arranged in a cross.

12. The system according to claim 11, wherein the distal portion (8c) of the piston (8) comprises a tip (16a), a sealing flange (16b) and a stop flange (16c), configured so that when the syringe (5) is pre-filled with the ferromagnetic agent and is assembled, the stop flange (16c) is placed level with the annular shoulder (11) of the cylinder (7), thus determining the thrust position of the piston (8).

13. The system according to any one of claims 10 to 12, wherein the gripping flange (9) comprises a recess (18) sized to be coupled to the open end (7a) of the cylinder (7) and a groove (19) surrounding the recess (18) and having a portion (19a) projecting towards the interior of the recess (18), the groove (19) being configured to be inserted into the annular shoulder (11).

14. An endoscopy kit, comprising an endoscopic system according to claim 3, or any one of claims 4 to 13 when dependent on claim 3, and a catheter.

15. An endoscopic kit, comprising an endoscopic system according to claim 1 or 2, wherein the kit further comprises:
- a glass vial filled with the ferromagnetic agent as a powder and a syringe pre-filled with saline solution (aqueous solution of NaCl and/or sodium citrate), or
- a glass vial with the suspension of ferromagnetic agent and an empty syringe.

## Patentansprüche

1. Endoskopiesystem, insbesondere für Koloskopie, umfassend:
- eine endoskopische Führung, insbesondere für Koloskope, umfassend ein rohrförmiges Führungselement (1) und einen Verankerungskopf (2), wobei das rohrförmige Führungselement (1) einen Längshohlraum (3) aufweist, wobei der Verankerungskopf (2) mit einem ausdehnbaren Behälter (4) versehen ist, der dazu konfiguriert ist, ein ferromagnetisches Mittel aufzunehmen, wobei der ausdehnbare Behälter (4) mit dem Längshohlraum (3) in Verbindung steht, wobei das Endoskopiesystem ferner ein ferromagnetisches Mittel umfasst, das dazu konfiguriert ist, in dem Längshohlraum beweglich zu sein, um den ausdehnbaren Behälter (4) zu füllen/zu entleeren,
**dadurch gekennzeichnet, dass** das ferromagnetische Mittel
eine wässrige Suspension von Carbonyleisen ist.

2. Endoskopiesystem nach Anspruch 1, ferner umfassend eine Magnetfeldquelle, die dazu konfiguriert ist, auf das ferromagnetische Mittel eine Haltekraft in Bezug auf eine Querzugrichtung auszuüben, wobei die Haltekraft größer als oder gleich 5 Newton bei einem Abstand zwischen 2 Zentimetern und 10 Zentimetern von dem ferromagnetischen Mittel ist.

3. System nach Anspruch 1 oder 2, ferner umfassend eine Spritze (5), die mit dem ferromagnetischen Mittel vorgefüllt wird.

4. System nach einem der Ansprüche 1 bis 3, wobei das Carbonyleisen eine Reinheit aufweist, die größer als oder gleich 97,5 % oder größer als oder gleich 99,5 % ist und das aus kugelförmigen Partikeln besteht, die das folgende Partikelgrößenprofil aufweisen:
D10 = 3-4,5 Mikron, oder etwa 4,1 Mikron
D50 = 5-10 Mikron, oder etwa 9,5 Mikron
D90 = 4-30 Mikron, oder etwa 26 Mikron.

5. System nach einem der Ansprüche 1 bis 4, wobei die wässrige Suspension von Carbonyleisen eine Gewichtsmenge an Carbonyleisen zwischen 45 % und 60 % oder zwischen 50 % und 55 % oder zwischen 52 % und 53 % bezogen auf das Gesamtgewicht der Suspension umfasst.

6. System nach einem der Ansprüche 1 bis 5, wobei die wässrige Suspension von Carbonyleisen Trinatriumcitrat in Gewichtsanteilen zwischen 2,5 % und 4,5 % bezogen auf das Gesamtgewicht der Suspension und/oder Natriumchlorid in Gewichtsanteilen zwischen 5 % und 8 % bezogen auf das Gesamtgewicht der Suspension umfasst.

7. System nach einem der Ansprüche 1 bis 6, wobei das rohrförmige Führungselement (1) ein einziges Lumen aufweist und einen Außendurchmesser (D) von weniger als 2,6 mm oder von etwa 2,5 mm aufweist sowie einen Innendurchmesser (d) des Lumens des rohrförmigen Führungselements (1) zwischen 1,6 mm und 1,75 mm oder von etwa 1,7 mm aufweist.

8. System nach einem der Ansprüche 1 bis 7, wobei der ausdehnbare Behälter (4) einen aufblasbaren Ballon umfasst oder daraus besteht und bemessen ist, sodass er ein Volumen von 22-28 ml oder von etwa 25 ml aufweist und in dem ausdehnbaren Zustand eine Länge zwischen 55 und 65 mm oder von etwa 60 mm sowie einen Querdurchmesser zwischen 20 und 30 mm oder von etwa 25 mm aufweist.

9. System nach Anspruch 3 oder nach einem der Ansprüche 4 bis 8, wenn diese von Anspruch 3 abhängig sind,
wobei die Spritze (5) einen Zylinder (7) umfasst, in den ein Kolben (8) gleitend eingesetzt wird, sowie einen abnehmbaren Greifflansch (9).

10. System nach Anspruch 9, wobei der Zylinder (7) ein offenes Ende (7a) umfasst, in das der Kolben (8) einsetzbar ist, und ein Verbindungsende (7b), das dem offenen Ende (7a) gegenüberliegt und mit einem Anschluss (10) für das rohrförmige Führungselement (1) versehen ist, wobei das offene Ende (7a) einen nach außen vorstehenden ringförmigen Absatz (11) umfasst und wobei der Anschluss (10) vom männlichen "Luer-Lock"-Typ ist und eine zylindrische Hülse (12) aufweisend eine Innengewinde (12a) sowie eine Düse (13) umfasst, die koaxial innerhalb der zylindrischen Hülse (12) angeordnet ist.

11. System nach Anspruch 9 oder 10, wobei der Kolben (8) einen Körper (8a) umfasst, der sich entlang einer Längsachse zwischen einem proximalen Abschnitt (8b) und einem distalen Abschnitt (8c) erstreckt und vorzugsweise aus einer Struktur (14) mit kreuzförmig angeordneten Flügeln besteht.

12. System nach Anspruch 11, wobei der distale Abschnitt (8c) des Kolbens (8) eine Spitze (16a), einen Dichtflansch (16b) und einen Anschlagflansch (16c) umfasst, die konfiguriert sind, sodass, wenn die Spritze (5) mit dem ferromagnetischen Mittel vorgefüllt wird und zusammengebaut wird, der Anschlagflansch (16c) auf Höhe des ringförmigen Absatzes (11) des Zylinders (7) angeordnet ist, indem die Vorschubposition des Kolbens (8) bestimmt wird.

13. System nach einem der Ansprüche 10 bis 12, wobei der Greifflansch (9) eine Ausnehmung (18) umfasst, die bemessen ist, um mit dem offenen Ende (7a) des Zylinders (7) gekoppelt zu werden, sowie eine Nut (19) umfasst, die die Ausnehmung (18) umgibt, die einen Abschnitt (19a) aufweist, der nach innen der Ausnehmung (18) vorsteht, wobei die Nut (19) dazu konfiguriert ist, in den ringförmigen Absatz (11) eingesetzt zu werden.

14. Endoskopie-Kit, umfassend ein Endoskopiesystem nach Anspruch 3 oder nach einem der Ansprüche 4 bis 13, wenn diese von Anspruch 3 abhängig sind, und ein Kathether.

15. Endoskopisches Kit, umfassend ein Endoskopiesystem nach Anspruch 1 oder 2, wobei das Kit ferner umfasst:
- eine Glasampulle, die mit dem ferromagnetischen Mittel in Pulverform gefüllt wird, und eine Spritze, die mit einer Salzlösung (wässrige Lösung von NaCl und/oder Natriumcitrat) vorgefüllt wird, oder
eine Glasampulle mit der Suspension des ferromagnetischen Mittels und eine leere Spritze.

## Revendications

1. Un système d'endoscopie, en particulier pour la coloscopie, comprenant :
un guide endoscopique, en particulier pour les coloscopes, comprenant un élément de guide tubulaire (1) et une tête d'ancrage (2), dans lequel l'élément de guide tubulaire (1) comprend une cavité longitudinale (3), la tête d'ancrage (2) étant pourvue d'un récipient expansible (4) configuré pour recevoir un agent ferromagnétique, le récipient expansible (4) étant en communication avec ladite cavité longitudinale (3), le système d'endoscopie comprenant en outre un agent ferromagnétique configuré pour être mobile dans ladite cavité longitudinale afin de remplir/vider le récipient expansible (4),
**caractérisé en ce que** l'agent ferromagnétique est une suspension aqueuse de fer carbonyle.

2. Le système d'endoscopie selon la revendication 1, comprenant en outre une source de champ magnétique configurée pour appliquer une force de rétention à l'agent ferromagnétique, par rapport à une direction de traction transversale, ladite force étant supérieure ou égale à 5 Newtons à une distance comprise entre 2 centimètres et 10 centimètres dudit agent ferromagnétique.

3. Le système selon la revendication 1 ou 2, comprenant en outre une seringue (5) préremplie dudit agent ferromagnétique.

4. Le système selon l'une quelconque des revendications 1 à 3, dans lequel ledit fer carbonyle présente une pureté supérieure ou égale à 97,5 %, ou supérieure ou égale à 99,5 %, et consiste en des particules sphériques ayant le profil granulométrique suivant :
D10 = 3-4,5 microns, ou environ 4,1 microns
D50 = 5-10 microns, ou environ 9,5 microns
D90 = 4-30 microns, ou environ 26 microns.

5. Le système selon l'une quelconque des revendications 1 à 4, dans lequel ladite suspension aqueuse de fer carbonyle comprend une quantité en poids de fer carbonyle comprise entre 45 % et 60 %, ou entre 50 % et 55 %, ou entre 52 % et 53 %, par rapport au poids total de la suspension.

6. Le système selon l'une quelconque des revendications 1 à 5, dans lequel ladite suspension aqueuse de fer carbonyle comprend du citrate de sodium tribasique en des quantités en poids comprises entre 2,5 % et 4,5 % par rapport au poids total de la suspension et/ou du chlorure de sodium en des quantités en poids comprises entre 5 % et 8 % par rapport au poids total de la suspension.

7. Le système selon l'une quelconque des revendications 1 à 6, dans lequel l'élément de guide tubulaire (1) comporte une seule lumière et présente un diamètre extérieur (D) inférieur à 2,6 mm, ou d'environ 2,5 mm, et un diamètre intérieur (d) de la lumière de l'élément de guide tubulaire (1) compris entre 1,6 mm et 1,75 mm, ou d'environ 1,7 mm.

8. Le système selon l'une quelconque des revendications 1 à 7, dans lequel le récipient expansible (4) comprend, ou consiste en, un ballon gonflable et est dimensionné de manière à avoir une capacité de 22-28 ml, ou d'environ 25 ml, et, lorsqu'il est en condition expansée, une longueur comprise entre 55 et 65 mm, ou d'environ 60 mm, et un diamètre transversal compris entre 20 et 30 mm, ou d'environ 25 mm.

9. Le système selon la revendication 3 ou l'une quelconque des revendications 4 à 8 lorsqu'elles dépendent de la revendication 3,
dans lequel la seringue (5) comprend un cylindre (7) dans lequel un piston (8) est inséré de manière coulissante, et une bride de préhension amovible (9).

10. Le système selon la revendication 9, dans lequel le cylindre (7) comprend une extrémité ouverte (7a), dans laquelle le piston (8) est insérable, et une extrémité de raccordement (7b), opposée à l'extrémité ouverte (7a), pourvue d'un connecteur (10) pour l'élément de guide tubulaire (1), dans lequel l'extrémité ouverte (7a) comprend un épaulement annulaire saillant vers l'extérieur (11) et dans lequel le connecteur (10) est du type mâle « Luer lock » et
comprend un manchon cylindrique (12) ayant un filetage interne (12a) et une buse (13) disposée coaxialement à l'intérieur du manchon cylindrique (12).

11. Le système selon la revendication 9 ou 10, dans lequel le piston (8) comprend un corps (8a) s'étendant le long d'un axe longitudinal entre une portion proximale (8b) et une portion distale (8c), et consiste de préférence en une structure (14) avec des ailettes disposées en croix.

12. Le système selon la revendication 11, dans lequel la portion distale (8c) du piston (8) comprend un embout (16a), une bride d'étanchéité (16b) et une bride d'arrêt (16c), configurées de sorte que, lorsque la seringue (5) est préremplie avec l'agent ferromagnétique et est assemblée, la bride d'arrêt (16c) est placée au niveau de l'épaulement annulaire (11) du cylindre (7), déterminant ainsi la position de poussée du piston (8).

13. Le système selon l'une quelconque des revendications 10 à 12, dans lequel la bride de préhension (9) comprend un évidement (18) dimensionné pour être accouplé à l'extrémité ouverte (7a) du cylindre (7) et une gorge (19) entourant l'évidement (18) et présentant une portion (19a) faisant saillie vers l'intérieur de l'évidement (18), la gorge (19) étant configurée pour être insérée dans l'épaulement annulaire (11).

14. Un kit d'endoscopie, comprenant un cathéter endoscopique selon la revendication 3 ou l'une quelconque des revendications 4 à 13 lorsqu'elle dépend de la revendication 3 et un cathéter.

15. Un kit endoscopique, comprenant un système d'endoscopie selon la revendication 1 ou 2, dans lequel le kit comprend en outre :
- un flacon en verre rempli de l'agent ferromagnétique sous forme de poudre et une seringue préremplie avec une solution saline (solution aqueuse de NaCl et/ou de citrate de sodium), ou
un flacon en verre contenant la suspension de l'agent ferromagnétique et une seringue vide.
